(19) Europäisches Patentamt
European Patent Office
Office européen des brevets

(11) Veröffentlichungsnummer: **0 377 846 A1**

(12) **EUROPÄISCHE PATENTANMELDUNG**

(21) Anmeldenummer: **89123023.7**

(22) Anmeldetag: **13.12.89**

(51) Int. Cl.5: **A61K 31/70, A61K 31/725**

Patentansprüche für folgende Vertragsstaaten:ES + GR.

(30) Priorität: **16.12.88 DE 3842362**

(43) Veröffentlichungstag der Anmeldung: **18.07.90 Patentblatt 90/29**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rösner, Manfred, Dr.**
**Unter den Buchen 7**
**D-6239 Eppstein/Taunus(DE)**
Erfinder: **Winkler, Irvin, Dr.**
**In den Eichen 40**
**D-6237 Liederbach(DE)**
Erfinder: **Meichsner, Christoph, Dr.**
**Bienerstrasse 30**
**D-6238 Hofheim am Taunus(DE)**

(54) **Pharmazeutische Kombinations-präparate enthaltend Nucleosid-Derivate und Pentosanpolysulfat sowie deren Herstellung und Verwendung.**

(57) Pharmazeutische Kombinationspräparate die mindestens ein Nucleosid-Derivat und Pentosanpolysulfat enthalten oder aus diesen bestehen, eignen sich zur Bekämpfung von Krankheiten, die durch Viren, insbesondere durch Retroviren verursacht worden sind.

EP 0 377 846 A1

# Pharmazeutische Kombinationspräparate enthaltend Nucleosid-Derivate und Pentosanpolysulfat sowie deren Herstellung und Verwendung

Die vorliegende Erfindung betrifft pharmazeutische Kombinationspräparate enthaltend Nucleosid-Derivate und Pentosanpolysulfat, sowie die Herstellung und Verwendung dieser Kombinationspräparate.

Es ist bekannt, daß bestimmte Nucleosid-Derivate, z.B. das 3′-Azido-3′-desoxythymidin (AZT) (Yarchoan et al.: The Lancet, 575-580 (1986)) zur Bekämpfung von Infektionen mit dem Human Immunodeficiency Virus (HIV) geeignet sind. Aus der Deutschen Offenlegungsschrift 36 01 136 ist weiterhin bekannt, daß bestimmte organische Polymere mit anorganischen anionischen Gruppen, insbesonder sulfatierte Polysaccharide zur Bekämpfung von Retrovirusinfektionen geeignet sind. Darüber hinaus wurde bereits vorgeschlagen, Viruserkrankungen durch die Verabreichung eines Kombinationspräparates, u.a. enthaltend ein Nucleosid-Derivat und ein sulfatiertes Polysaccharid, zu bekämpfen (vgl. EP 0 270 317).

Überraschenderweise wurde nun gefunden, daß ein Kombinationspräparat, das bestimmte Nucleosid-Derivate und ein Pentosanpolysulfat enthält eine besonders ausgeprägte Wirksamkeit gegen Virusinfektionen, insbesondere gegen Infektionen mit Retroviren hat.

Erfindungsgegenstand ist demzufolge ein pharmazeutisches Kombinationspräparat, das aus mindestens einer Verbindung der Gruppe A) Nucleosid-Derivate der Formeln I - V

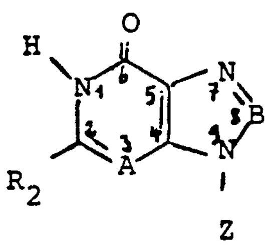 (I), 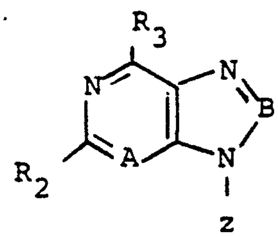 (II),

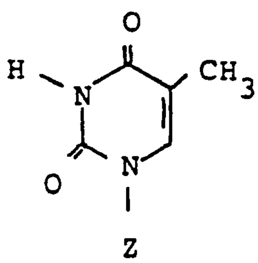 (III), 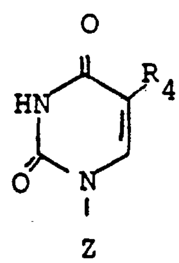 (IV), 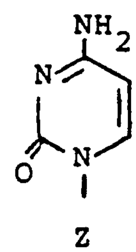 (V)

in denen

A und B unabhängig voneinander CH oder N sind,

$R_2$ H oder $NH_2$ ist,

$R_3$ H, $NH_2$ oder SH ist,

$R_4$ H, F oder Cl ist und

Z ein Substituent der Formel VI

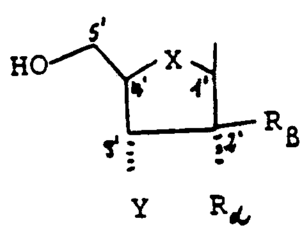 (VI)

ist, in der

X O, $CH_2$ oder $CR_\alpha R_\beta$,

Y $N_3$, $NH_2$, F, Cl, H oder $C_1$-$C_4$-Alkoxy,

$R_\alpha$ H oder F,

$R_\beta$ H, F oder OH oder Y und $R_\alpha$ wenn $R_\beta$ = H eine Doppelbindung bedeuten,

oder Z eine Gruppe der Formel VII ist

$$\begin{array}{l} | \\ (CH_2)_m \\ | \\ (O)_n - (CRR')_p - CH_2 - OH \end{array} \qquad (VII)$$

in der R und R' unabhängig voneinander H, OH, $CH_2OH$, $N_3$, $NH_2$, F, Cl oder an benachbarten C-Atomen zusammen eine Doppelbindung bedeuten,
wobei m = 0 oder 1, n = 0 oder 1 und p = 2, 3 oder 4 ist, und die Komponente
B) mindestens ein Pentosanpolysulfat und/oder mindestens eines seiner Salze enthält oder aus diesen Komponenten besteht. Nachfolgend sollen unter dem Begriff Pentosanpolysulfat gleichfalls dessen Salze verstanden werden.

Bevorzugte pharmazeutische Kombinationspräparate enthalten mindestens ein Nucleosid-Derivat der oben beschriebenen Formeln I - V, in denen die Verbindung der Gruppe A) ein Nucleosid-Derivat der Formeln I, II, III, IV oder V, wie zuvor abgebildet, ist, in denen
A N,
B CH,
$R_2$ für die Verbindung der Formel I $NH_2$ oder H und für die Verbindung der Formel II H ist, während $R_3$ $NH_2$ bedeutet, $R_4$ H oder Cl bedeutet und in denen Z ein Substituent der oben beschriebenen Formel VI ist, in dem
X O,
Y $N_3$, F oder H,
$R_\alpha$ H und
$R_\beta$ H oder OH bedeutet.

Von diesen Nucleosid-Derivaten sind wiederum bevorzugt 3'-Azido-3'-deoxythymidin, 3'-Azido-2',3'-dideoxy-uridin, -cytidin, -guanosin, -adenosin, -inosin, -diaminopurin, 3'-Fluor-3'-deoxythymidin, 3'-Fluor-2',3'-dideoxy-uridin, -cytidin, -guanosin, -adenosin, -inosin, -diaminopurin, 3'-Fluor-2',3'-dideoxy-5-chloruridin, 3'-Deoxythymidin, 2',3'-Dideoxy-uridin, -cytidin, -guanosin, -adenosin, -inosin, -diaminopurin sowie 3'-Deoxy-2',3'-didehydrothymidin, 2',3'-Dideoxy-2',3'-didehydro-uridin, -cytidin, -guanosin, -adenosin, -inosin, -diaminopurin, insbesondere 3'-Azido-3'-deoxythymidin, 3'-Azido-2',3'-dideoxy-uridin, -guanosin,
3'-Fluor-3'-deoxythymidin, 3'-Fluor-2',3'-dideoxy-uridin, -guanosin, 3'-Fluor-2',3'-dideoxy-5-chloruridin,
3'-Deoxythymidin, 2',3'-Dideoxy-cytidin, -guanosin, -adenosin,
-inosin, -diaminopurin und
3'-Deoxy-2',3'-didehydrothymidin, 2',3'-Dideoxy-2',3'-didehydro-uridin.

Ganz besonders bevorzugt sind
3'-Azido-3'-deoxythymidin,
3'-Fluor-3'-deoxythymidin,
3'-Fluor-2',3'-dideoxy-5-chloruridin,
3'-Deoxythymidin, 2',3'-Dideoxy-cytidin, -adenosin, -inosin,
3'-Deoxy-2',3'-didehydrothymidin.

Das in dem erfindungsgemäßen pharmazeutischen Kombinationspräparat enthaltene Pentosanpolysulfat (Synonyme Xylanhydrogensulfat, Xylanpolysulfat; vgl. Merck Index, 10. Auflage, Merck & Co. Inc., Rahway N.J. (1983), U.S.A.) kann unterschiedliche Molekülparameter aufweisen.

Ein bevorzugtes Pentosanpolysulfat weist ein mittleres Molekulargewicht von ca. 1000 Dalton bis ca. 12000 Dalton sowie einen Sulfatierungsgrad der Polysaccharid-OH-Gruppen von ca. 20 % bis ca. 100 % auf.

Ein besonders bevorzugtes Pentosanpolysulfat hat ein mittleres Molekulargewicht von 4500 - 7000 Dalton, insbesondere von ca. 6000 Dalton und einen Sulfatierungsgrad von ca. 75 % - 95 %, insbesondere von ca. 90 %. Ein außerordentlich geeignetes Pentosanpolysulfat ist das Pentosanpolysulfat SP 54 der Firma bene Chemie GmbH, 8000 München 71, Herterichstraße 1.

Das sulfatierte Polysaccharid kann als freie Säure, d.h. mit der funktionellen Gruppierung $OSO_3H$, oder als physiologisch verträgliches Salz vorliegen. Von den physiologisch verträglichen Salzen sind insbesondere die Ammonium- und Alkalimetallsalze bevorzugt. Ganz besonders bevorzugt ist das Natriumsalz.

Das erfindungsgemäße pharmazeutische Kombinationspräparat kann Verbindungen der Gruppe A) und Pentosanpolysulfat in beliebigem Verhältnis zueinander enthalten. Bevorzugt ist ein Gewichts-Verhältnis von Nucleosid-Derivat(en) zu Pentosanpolysulfat von 1:100 bis 100:1, besonders bevorzugt von 10:1 bis 1:10.

Die erfindungsgemäßen pharmazeutischen Kombinationspräparate sind wirksam gegen eine Reihe humanpathogener Viren, wie z.B. Herpesviren, besondere Bedeutung besitzt jedoch ihre Wirksamkeit gegen Retroviren, insbesondere gegen das HIV.

Zur Behandlung bzw. Prophylaxe von Erkrankungen, die durch Viren - insbesondere durch Retroviren - verursacht werden, können die erfindungsgemäßen Kombinationspräparate auf unterschiedliche Weise verabreicht werden. Z.B. können sie intravenös, intramuskulär, intraperitoneal, subkutan, als Dauertropfinfusion, rektal oder oral verabreicht werden. Bei akuten Krankheitszuständen ist die Verabreichung als Dauertropfinfusion vorzuziehen. Zur Dauermedikation ist z.B. die orale Verabreichung angezeigt. Die Verabreichung der einzelnen Komponenten, d.h. des bzw. der Nucleosid-Derivate und des Pentosanpolysulfats kann gleichzeitig oder mit zeitlicher Verzögerung, z.B. im Abstand von bis zu mehreren Stunden erfolgen; zu bevorzugen ist jedoch die gleichzeitige Verabreichung, besonders bevorzugt die gleichzeitige Verabreichung in einer Dosierungseinheit.

Die Herstellung der erfindungsgemäßen pharmazeutischen Kombinationspräparate erfolgt, indem mindestens ein Nucleosid-Derivat und Pentosanpolysulfat gegebenenfalls mit weiteren Hilfs- und/oder Trägerstoffen in eine geeignete Darreichungsform gebracht werden. Die Hilfs- und Trägerstoffe stammen aus der Gruppe der Trägermittel, Konservierungsstoffe und anderer üblicher Hilfsstoffe.

Z.B. können für orale Darreichungsformen Hilfsstoffe wie Stärken, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente des Kombinationspräparates, ein Retardierungsmittel zuzusetzen, gegebenenfalls in Form von permeablen Membranen, wie z.B. solche auf Cellulose- oder Polystyrolharzbasis, oder Ionenaustauscher.

Die anzuwendende Dosierung der erfindungsgemäßen Kombinationspräparate ist abhängig von verschiedenen Faktoren wie Darreichungsform des Medikaments und Zustand, Gewicht und Art der Erkrankung des Patienten. Eine Tagesdosis von ca. 1500 mg Nucleosid-Derivat und ca. 5000 mg eines Pentosanpolysulfats sollte jedoch nur kurzzeitig überschritten werden. Bevorzugt sind ca. 10 bis 1200 mg Nucleosid-Derivat und ca. 10 bis 2500 mg Pentosanpolysulfat jeweils als Tagesdosis für einen Menschen von ca. 70 kg Körpergewicht. Darüber hinaus ist es zweckmäßig, für einzelne konkrete Wirkstoffkombinationen sowohl Zusammensetzung als auch Dosierung z. B. durch Bestimmung der Bioverfügbarkeit in Versuchen zu optimieren.

Die Verabreichung der Tagesdosis des erfindungsgemäßen Kombinationspräparates kann in Form einer einzelnen Verabreichung oder in mehreren kleinen Dosen erfolgen. Bevorzugt ist die Verabreichung in 3 - 8 Dosen pro Tag. In manchen Fällen kann eine kontinuierliche Zuführung des Kombinationspräparates z.B. in Form einer Dauertropfinfusion angezeigt sein.

Die Herstellung der für die erfindungsgemäßen Kombinationspräparate notwendigen Nucleosid-Derivate ist literaturbekannt bzw. kann in Analogie zu bekannten Verfahren durchgeführt werden (vgl. z.B. Nucleic Acid Chemistry, Parts I - III, edited by Leroy B. Townsend, R. Stuard Tipson, John Wiley & Sons, (1978) New York, Chichester, Brisbane, Toronto, Singapore).

Bestimmte für das erfindungsgemäße Kombinationspräparat benötigte Pentosanpolysulfate sind kommerziell erhältlich. Pentosanpolysulfate mit einem speziellen Sulfatierungsgrad lassen sich z.B. durch Sulfatierung von Pentosan mit Schwefelsäure oder einem ihrer aktivierten Komplexe herstellen (vgl. z.B. Deutsche Offenlegungsschrift P 37 25 554.1, EP 0 270 317 oder Schweizer Patent 293 566).

Die Isolierung von Pentosanen bzw. Pentosanpolysulfaten mit einem bestimmten mittleren Molekulargewicht ist durch bekannte Trennverfahren, insbesondere chromatographische Trennverfahren möglich.

Durch die nachfolgenden Zubereitungsbeispiele soll die Erfindung näher erläutert werden:

**Beispiel 1:**

Tabletten, die für die orale Verabreichung geeignet sind und die nachfolgenden Bestandteile enthalten, werden auf an sich bekannte Weise hergestellt, indem man Wirk- und Hilfsstoffe granuliert und anschließend zu Tabletten verpreßt. Diese Tabletten eignen sich zur antiviralen Behandlung in einer Dosis von einer die gewünschte Dosierung gewährleistende Anzahl an Tabletten 3 - 6 mal täglich.

| Bestandteile (pro Tablette) | Gewicht |
|---|---|
| Pentosanpolysulfat, Na-Salz<br>Mittleres Molekulargew. 6000 Dalton<br>Sulfatierungsgrad 90 % | 150 mg |
| 3′-Azido-3′-deoxythymidin | 100 mg |
| Milchzucker | 150 mg |
| Maisstärke | 50 mg |
| Talkum | 6 mg |
| kolloidales Siliziumdioxid | 6 mg |
| Magnesiumstearat | 4 mg |

**Beispiel 2:**

Kapseln, die für die orale Verabreichung geeignet sind, enthalten die nachfolgend genannten Bestandteile und können auf an sich bekannte Weise hergestellt werden, indem man Wirk- und Hilfsstoffe vermischt und in Gelatinekapseln abfüllt. Diese Kapseln dienen zur antiviralen Behandlung in einer Dosis von einer Kapsel 2 - 4mal täglich.

| Bestandteile (pro Kapsel) | Gewicht |
|---|---|
| Pentosanpolysulfat, Na-Salz<br>mittleres Molekulargew. 6000 Dalton,<br>Sulfatierungsgrad 90 % | 150 mg |
| 3′-Azido-3′-deoxythymidin | 100 mg |
| Milchzucker | 200 mg |
| Talkum | 10 mg |
| kolloidales Siliziumdioxid | 10 mg |

Beispiel 3:

Wirkstofflösungen, die zur Injektion geeignet sind, enthalten die nachfolgend genannten Bestandteile und können auf an sich bekannte Weise hergestellt werden, indem die Stoffe miteinander vermischt und in sterile Ampullen abgefüllt werden. Die Injektionslösungen dienen zur antiviralen Behandlung in einer Dosis von 1 - 2 Injektionseinheiten (1 Injektionseinheit = 1 Ampulle) pro Tag.

| Bestandteile (pro Ampulle) | Gewicht |
|---|---|
| Pentosanpolysulfat, Na-Salz<br>mittleres Molekulargewicht 6000 Dalton,<br>Sulfatierungsgrad 90 % | 150 mg |
| 3′-Azido-3′-deoxythymidin | 100 mg |
| Natriumchlorid | 50 mg |
| Methylparaben | 5 mg |
| steriles Wasser | 5 g |

**Wirksamkeitstests**

Prüfung von Präparaten gegen HIV in der Zellkultur

Methodenbeschreibung

**Medium:**

RPMI pH 6,8
Komplettes Medium enthält zusätzlich 20 % foetales Kälberserum und 20 IU/ml rekombinantes Interleukin 2.

**Zellen:**

Aus frischem Spenderblut mittels Ficoll[R]-Gradientenzentrifugation isolierte Lymphozyten werden unter Zusatz von 2 µg/ml Phytohämagglutinin (Wellcome) in komplettem Medium 36 h bei 37° C unter 5 % $CO_2$ kultiviert. Die Zellen werden nach Zusatz von 10 % DMSO bei einer Zelldichte von $5 \times 10^6$ eingefroren und in flüssigem Stickstoff gelagert. Für den Versuch werden die Zellen aufgetaut, in RPMI-Medium gewaschen und in komplettem Medium 3 - 4 Tage kultiviert.

**Ansatz:**

Die Prüfpräparate werden in komplettem Medium gelöst, üblicherweise in einer Konzentration von 1,2 mg/ml. In 24er Multiwell-Schalen werden jeweils 0,5 ml komplettes Medium gegeben. Nach Zusatz von 0,1 ml des gelösten Präparates wird durch Übertragung von jeweils 0,1 ml das Präparat in einer geometrischen Verdünnungsreihe, Faktor 5, verdünnt. Zu allen Ansätzen werden 0,4 ml einer Suspension mit $5 \times 10^5$ Lymphozyten pro ml gegeben.

Die Ansätze werden mit 0,1 ml eines Überstandes von HIV 1 infizierten Lymphozyten versetzt, der so eingestellt ist, daß im Ansatz der cytopathogene Effekt nach 3 - 4 Tagen erkennbar ist.
Die Multiplizität der infektiösen Einheit beträgt hierbei 0,01 - 0,02.

Der Versuchsansatz enhält damit folgende Komponenten in komplettem Medium

0,5 ml Präparat 200 µg/ml bis 1,6 µg/ml
0,4 ml Zellen ca. $1 \times 10^6$/ml
0,1 ml HIV     1:20-1:50
1,0 ml

Die Versuchsansätze enthalten außerdem präparatfreie Infektionskontrollen bzw. Standardpräparate, z.B. 3′-Azido-3′-deoxythymidin 0,2 µg/ml oder Pentosanpolysulfat 25 µg/ml.

**Auswertung:**

Die Auswertung erfolgt durch mikroskopische Betrachtung der Ansätze am 4. Tage nach Infektion.
Anhand der virusbedingten Syncytienbildung wird die Virusvermehrung bestimmt und die minimale Hemmkonzentration (MHK) bestimmt.

I. Hemmung der HIV induzierten Syncytienbildung in Kulturen menschlicher Lymphozyten durch Pentosanpolysulfat (Natriumsalz, Sulfatierungsgrad ca. 80 - 90 %, mittleres Molekulargewicht 6000 Dalton) in Kombination mit AZT und durch Dextransulfat (Natriumsalz, Sulfatierungsgrad ca. 60 - 90 %, mittleres Molekulargewicht 8000 Dalton) in Kombination mit AZT
Die Ergebnisse dieses Tests zeigt Tabelle 1.

Tabelle 1

| | MHK-Wert µg/ml | |
|---|---|---|
| 3'-Azido-3'-deoxythymidin µg/ml | Dextransulfat | Pentosanpolysulfat |
| 0 | 100 | 25 |
| 0,012 | 25 | 12,5 |
| 0,025 | < 12,5 | < 1,6 |
| 0,05 | < 12,5 | < 1,6 |
| 0,1 | < 12,5 | < 1,6 |
| MHK-Wert: 0,1 µg/ml | | |

Die in Tabelle 1 aufgezeichneten Daten belegen die hervorragende Wirksamkeit von Pentosanpolysulfat in Kombination mit AZT.

II Hemmung der HIV induzierten Syncytienbildung in Kulturen menschlicher Lymphozyten durch Pentosanpolysulfat (Natriumsalz, Sulfatierungsgrad 90%, mittleres Molekulargewicht 6000 Dalton) in Kombination mit Nucleosid-Analoga

Die Ergebnisse dieses Tests zeigt Tabelle 2:

**Tabelle 2**

| | | MHK-Wert (µg/ml) von Pentosanpolysulfat |
|---|---|---|
| 3'-Azido-3'-deoxythymidin | | |
| MHK-Wert: | 0,1 µg/ml | |
| | 0 | 25 |
| | 0,012 | 12,5 |
| | 0,025 | < 1,6 |
| | 0,05 | < 1,6 |
| | 0,1 | < 1,6 |
| 3'-Azido-2',3'-dideoxyguanosin | | |
| MHK-Wert: | >2 µg/ml | |
| | 0 | 25 |
| | 0,25 | 25 |
| | 0,5 | 25 |
| | 1,0 | 12,5 |
| | 2,0 | 12,5 |

| | | MHK-Wert (µg/ml) von Pentosanpolysulfat |
|---|---|---|
| 3'-Fluoro-2',3'-dideoxythymidin | | |
| MHK-Wert | 0,05 µg/ml | |
| | 0 | 25 |
| | 0,013 | 25 |
| | 0,025 | > 25 |
| | 0,05 | < 3,1 |
| | 0,1 | < 3,1 |
| 2',3'-Dideoxycytidin | | |
| MHK-Wert: | 0,5 µg/ml | |
| | 0 | 12,5 |
| | 0,125 | 6,25 |
| | 0,25 | < 3,1 |
| | 0,5 | < 3,1 |
| | 1,0 | < 3,1 |

Die in Tabelle 2 aufgetragenen Ergebnisse zeigen, daß Pentosanpolysulfat in Kombination mit verschiedenen Nucleosid-Derivaten hervorragende antivirale Aktivität besitzt.

## Ansprüche

1. Pharmazeutisches Kombinationspräparat, dadurch gekennzeichnet, daß es mindestens eine Verbindung der Gruppe

A) Nucleosid-Derivate der Formeln I - V

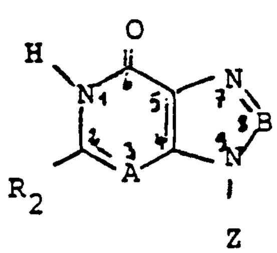 (I),

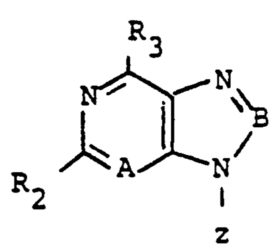 (II),

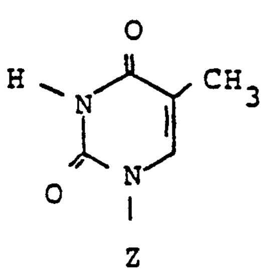 (III),

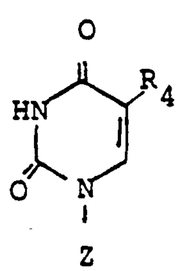 (IV),

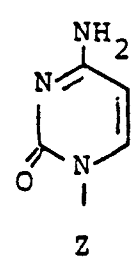 (V)

in denen
A und B unabhängig voneinander CH oder N sind,
$R_2$ H oder $NH_2$ ist,
$R_3$ H, $NH_2$ oder SH ist,
$R_4$ H, F oder Cl ist
und Z ein Substituent der Formel VI

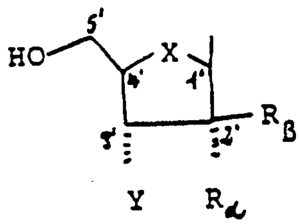

(VI)

ist, in der
X 0, $CH_2$ oder $CR_\alpha R_\beta$,
Y $N_3$, $NH_2$, F, Cl, H oder $C_1$-$C_4$-Alkoxy,
$R_\alpha$ H oder F,
$R_\beta$ H, F oder OH oder Y und $R_\alpha$ wenn $R_\beta$ = H eine Doppelbindung bedeuten, oder Z eine Gruppe der Formel VII ist,

$$\begin{matrix}(CH_2)_m \\ (O)_n\end{matrix} - (CRR')_p - CH_2 - OH \qquad (VII)$$

in der R und R′ unabhängig voneinander H, OH, $CH_2OH$, $N_3$, $NH_2$, F, Cl oder an benachbarten C-Atomen zusammen eine Doppelbindung bedeuten,
wobei m = 0 oder 1, n = 0 oder 1 und p = 2, 3 oder 4 ist und die Komponente
B) mindestens ein Pentosanpolysulfat und/oder mindestens eines seiner Salze enthält oder aus diesen besteht.

2. Pharmazeutisches Kombinationspräparat gemäß Anspruch 1, dadurch gekennzeichnet, daß die Verbindung der Gruppe A) ein Nucleosid-Derivat der Formeln I, II, III, IV oder V gemäß Anspruch 1 ist, in denen
A N,
B CH,
$R_2$ für die Verbindung der Formel I $NH_2$ oder H und für die Verbindung der Formel II H ist, während
$R_3$ $NH_2$ und $R_4$ H oder Cl bedeutet
und in denen Z ein Substituent der Formel VI ist, in dem
X O
Y $N_3$, F oder H,
$R_\alpha$ H und
$R_\beta$ H oder OH bedeutet.

3. Pharmazeutisches Kombinationspräparat gemäß Anspruch 1 oder 2, dadurch gekennzeichnet, daß die Verbindung der Gruppe A) ein Nucleosid-Derivat ausgewählt aus folgenden Verbindungen ist 3′-Azido-3′-deoxythymidin, 3′-Azido-2′,3′-dideoxy-uridin, -guanosin,
3′-Fluor-3′-deoxythymidin, 3′-Fluor-2′,3′-dideoxyuridin, -guanosin, 3′-Fluor-2′,3′-dideoxy-5-chloruridin, 3′-Deoxythymidin, 2′,3′-Dideoxy-cytidin, -guanosin, -adenosin, -inosin, -diaminopurin,
3′-Deoxy-2′,3′-didehydrothymidin, 2′,3′-Dideoxy-2′,3′-didehydro-uridin.

4. Pharmazeutisches Kombinationspräparat nach einem oder mehreren der Ansprüche 1 - 3, dadurch gekennzeichnet, daß das Pentosanpolysulfat der Komponente B) ein mittleres Molekulargewicht von ca. 1000 bis ca. 12000 Dalton und einen Sulfatierungsgrad der Polysaccharid-OH-Gruppen von 20 bis 100 % aufweist.

5. Pharmazeutisches Kombinationspräparat nach einem oder mehreren der Ansprüche 1 - 4, dadurch gekennzeichnet, daß das Pentosanpolysulfat der Komponente B) ein mittleres Molekulargewicht von ca.

3500 - 6500 Dalton und einen Sulfatierungsgrad von 75 - 95 % aufweist.

6. Pharmazeutisches Kombinationspräparat nach einem oder mehreren der Ansprüche 1 - 5, dadurch gekennzeichnet, daß das Gewichtsverhältnis der Verbindung(en) der Gruppe A) und der Komponente B) zwischen 100:1 und 1:100 liegt.

7. Pharmazeutisches Kombinationspräparat nach einem oder mehreren der Ansprüche 1 - 6 zur Behandlung bzw. Prophylaxe von Infektionskrankheiten, die durch Viren hervorgerufen werden.

8. Pharmazeutisches Kombinationspräparat nach einem oder mehreren der Ansprüche 1 - 7 zur Behandlung bzw. zur Prophylaxe von Infektionskrankheiten, die durch Retroviren hervorgerufen werden.

9. Verwendung eines pharmazeutischen Kombinationspräparates gemäß einem oder mehreren der Ansprüche 1 - 7 zur Behandlung bzw. zur Prophylaxe von Infektionskrankheiten, die durch Viren hervorgerufen werden.

10. Verfahren zur Herstellung eines pharmazeutischen Kombinationspräparates nach einem oder mehreren der Ansprüche 1 bis 7, dadurch gekennzeichnet, daß man mindestens eine Verbindung der Gruppe A) und die Komponente B) ein Pentosanpolysulfat gegebenenfalls mit üblichen Hilfs- und/oder Trägerstoffen in eine geeignete Darreichungsform bringt.

Patentansprüche für folgende Vertragsstaaten: ES, GR

1. Verfahren zur Herstellung eines pharmazeutisches Kombinationspräparates, dadurch gekennzeichnet, daß man mindestens eine Verbindung der Gruppe

A) Nucleosid-Derivate der Formeln I - V

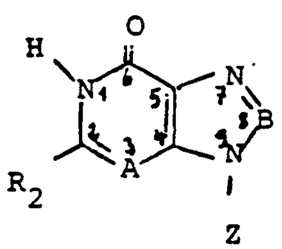 (I),

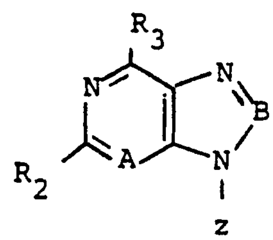 (II),

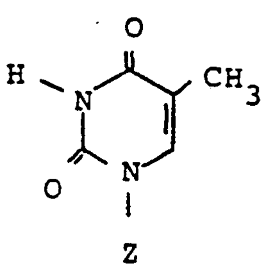 (III),

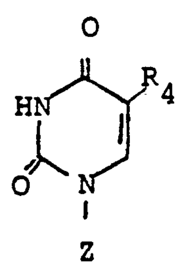 (IV),

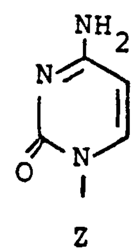 (V)

in denen

A und B unabhängig voneinander CH oder N sind,

$R_2$ H oder $NH_2$ ist,

$R_3$ H, $NH_2$ oder SH ist,

$R_4$ H, F oder Cl ist und Z ein Substituent der Formel VI

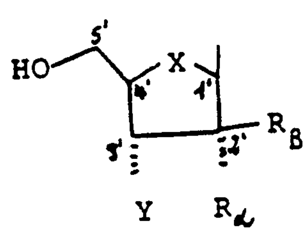 (VI)

ist, in der

X O, $CH_2$ oder $CR_\alpha R_\beta$,

Y $N_3$, $NH_2$, F, Cl, H oder $C_1$-$C_4$-Alkoxy, $R_\alpha$H oder F,

$R_\beta$ H, F oder OH oder Y und $R_\alpha$ wenn $R_\beta$ = H eine Doppelbindung bedeuten, oder Z eine Gruppe der Formel VII ist,

$$\begin{array}{l} | \\ (CH_2)_m \\ | \\ (O)_n - (CRR')_p - CH_2 - OH \end{array} \qquad (VII)$$

in der R und R′ unabhängig voneinander H, OH, $CH_2OH$, $N_2$, $NH_2$, F, Cl oder an benachbarten C-Atomen zusammen eine Doppelbindung bedeuten, wobei m = 0 oder 1, n = 0 oder 1 und p = 2, 3 oder 4 ist. und die Komponente

B) mindestens ein Pentosanpolysulfat und/oder mindestens eines seiner Salze enthält oder aus diesen besteht, gegebenenfalls mit Hilfs- und/oder Trägerstoffen in eine geeignete Darreichungsform bringt.

2. Verfahren gemäß Anspruch 1, dadurch gekennzeichnet, daß die Verbindung der Gruppe A) ein Nucleosid-Derivat der Formeln I, II, III, IV oder V gemäß Anspruch 1 ist, in denen

A N,
B CH,
$R_2$ für die Verbindung der Formel I $NH_2$ oder H und für die Verbindung der Formel II H ist, während
$R_3$ $NH_2$ und $R_4$ H oder Cl bedeutet und in denen Z ein Substituent der Formel VI ist, in dem
X O
Y $N_3$, F oder H,
$R_\alpha$ H und
$R_\beta$ H oder OH bedeutet.

3. Verfahren gemäß Anspruch 1 oder 2, dadurch gekennzeichnet, daß die Verbindung der Gruppe A) ein Nucleosid-Derivat ausgewählt aus folgenden Verbindungen ist 3′-Azido-3′-deoxythymidin, 3′-Azido-2′,3′-dideoxy-uridin, -guanosin, 3′-Fluor-3′-deoxythymidin, 3′-Fluor-2′,3′-dideoxy-uridin, -guanosin, 3′-Fluor-2′,3′-dideoxy-5-chloruridin, 3′-Deoxythymidin, 2′,3′-Dideoxy-cytidin, -guanosin, -adenosin, inosin, -diaminopurin, 3′-Deoxy-2′,3′-didehydrothymidin, 2′,3′-Dideoxy-2′,3′-didehydro-uridin.

4. Verfahren gemäß einem oder mehreren der Ansprüche 1 - 3, dadurch gekennzeichnet, daß das Pentosanpolysulfat der Komponente B) ein mittleres Molekulargewicht von ca. 1000 bis ca. 12000 Dalton und einen Sulfatierungsgrad der PolysaccharidOH-Gruppen von 20 bis 100 % aufweist.

5. Verfahren gemäß einem oder mehreren der Ansprüche 1 - 4, dadurch gekennzeichnet, daß das Pentosanpolysulfat der Komponente B) ein mittleres Molekulargewicht von ca. 3500 - 6500 Dalton und einen Sulfatierungsgrad von 75 -95 % aufweist.

6. Verfahren gemäß einem oder mehreren der Ansprüche 1 - 5, dadurch gekennzeichnet, daß das Gewichtsverhältnis der Verbindung(en) der Gruppe A) und der Komponente B) zwischen 100:1 und 1:100 liegt.

7. Pharmazeutisches Kombinationspräparat hergestellt gemäß einem oder mehreren der Ansprüche 1 - 6 zur Behandlung bzw. Prophylaxe von Infektionskrankheiten, die durch Viren hervorgerufen werden.

8. Pharmazeutisches Kombinationspräparat hergestellt gemäß einem oder mehreren der Ansprüche 1 - 6 zur Behandlung bzw. zur Prophylaxe von Infektionskrankheiten, die durch Retroviren hervorgerufen werden.

9. Verwendung eines pharmazeutischen Kombinationspräparates hergestellt gemäß einem oder mehreren der Ansprüche 1 - 6 zur Behandlung bzw. zur Prophylaxe von Infektionskrankheiten, die durch Viren hervorgerufen werden.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 89 12 3023

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,P | BIOLOGICAL ABSTRACTS/RRM, zusammenfassings Nr. 37083153; R. ANAND et al.:"Sodium pentosan polysulfate SP-54 an anti-HIV agent also exhibits lymphocyte proliferative activity and synergism with AZT" & CANADA. ILLUS. PAPER. ISBN 0-662-56670-X. 1989, Band 0, Nr. 0, Seite 635 | 1-3,7, 8 | A 61 K 31/70<br>A 61 K 31/725 |
| | -- | | |
| A,D | EP-A-0 270 317 (KABUSHIKI KAISHA VENO SEIYAKU OYO KENKYUJO)<br>* Ansprüche * | 1-8, 10 | |
| | -- | | |
| A,D | DE-A-3 725 554 (HOECHST AG)<br>* Ansprüche * | 1-8, 10 | **RECHERCHIERTE SACHGEBIETE (Int. Cl.4)**<br>A 61 K |
| | -- ./. | | |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8,10

Unvollständig recherchierte Patentansprüche:

Nicht recherchierte Patentansprüche: 9

Grund für die Beschränkung der Recherche:

Verfahren zur chirurgischen oder therapeutischen Behandlung des menschlichen oder tierischen Körpers (Siehe Art. 52(4) des Europäischen Patentübereinkommens).

| Recherchenort | Abschlußdatum der Recherche | Prüfer |
|---|---|---|
| DEN HAAG | 22-03-1990 | LEHERTE |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82

Europäisches Patentamt

**EUROPÄISCHER TEILRECHERCHENBERICHT**

Nummer der Anmeldung
EP 89 12 3023
-2-

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| A | DIALOG 07327008, Exerpta Medica, 89040776;<br>L. BIESERT et al.: "Inhibition of HIV and virus replication by polysulphated polyxylan: HOE/BAY 946, a new antiviral compound"<br>& AIDS (GB), Band 2/6, 1988, Seiten 449-457<br>* Zusammenfassung *<br>---- | 1-8,10 | |
| | | | RECHERCHIERTE SACHGEBIETE (Int. Cl.4) |

EPA Form 1505.3 06.78